# EUROPEAN PATENT APPLICATION

(11) **EP 4 382 159 A1**
(43) Date of publication of application: **12.06.2024**
(21) Application number: 22913400.2
(22) Date of filing: 17.08.2022
(51) Int. Cl.: A61M 25/08

(54) **DETACHMENT MECHANISM AND DELIVERY CATHETER**

(30) Priority: 29.12.2021 CN 202111681706
(71) Applicant: Shanghai Trulive Medtech Co., Ltd., Shanghai 201206 (CN); Jiangsu Trulive Medtech Co., Ltd., Nantong, Jiangsu 226400 (CN)
(72) Inventor: SUN, Yantao, Shanghai 201206 (CN); ZHAO, Jing, Shanghai 201206 (CN); SUN, Yunyan, Shanghai 201206 (CN); ZHANG, Wei, Shanghai 201206 (CN); XU, Haoran, Shanghai 201206 (CN); HE, Dong, Shanghai 201206 (CN); ZHANG, Haijun, Shanghai 201206 (CN)
(74) Representative: Nordmeyer, Philipp Werner
(86) International application number: PCT/CN2022/112967
(87) International publication number: WO 2023/124115

(57) **Abstract**

A detachment mechanism and a delivery catheter are used to enable delivery and detachment of an implant (1). The detachment mechanism includes a detacher (10), a first control member (20) and a second control member (30). A distal end of the detacher (10) is configured for engagement of the implant (1) therewith. The detacher (10) defines a through hole (11). The first control member (20) and the second control member (30) are detachably engaged with or directly joined to each other. When distal ends of the first control member (20) and the second control member (30) are inserted through the through hole (11) and engaged with each other, the first control member (20) and the second control member (30) are locked against the detacher (10). When the first control member (20) is pulled and removed from the through hole (11), the first control member (20) and the second control member (30) are detached from the detacher (10). In this way, relocation of the detacher (10) is allowed, resulting in improved delivery accuracy.

## Description

### TECHNICAL FIELD

The present invention relates to the field of medical devices and, in particular, to a detachment mechanism and a delivery catheter.

### BACKGROUND

Advances in our living and healthcare conditions are increasing the average human age every year. This is accompanied by a rise of incidence of valvular heart disease on a year-by-year basis. The intricate and complex anatomy of the human body necessitates different types of valves for treatment of valvular heart disease. Minimally invasive interventional surgery provides an option for patients who cannot afford thoracotomy. Various minimally invasive interventional procedures involve delivery of through different vascular accesses, accurate placement and detachment, of implants. Due to dimensional limitations of human blood vessels, compared with single-layer catheters, multi-layer delivery catheters for this purpose must have smaller component tubes, making them more difficult to fabricate. Moreover, gap(s) between the component tubes is/are desired to be minimized, necessitating larger pushing forces for their delivery. For reasons such as dimensional limitation and excessively large required pushing forces, it is difficult for currently available delivery catheters for this purpose to satisfactorily meet the requirements for delivery and detachment as their coupling designs are suboptimal and associated with easy deformation or even breakage at a joint.

Due to dimensional limitations of blood vessels, such a delivery catheter is usually equipped with a miniature detachment mechanism, which usually remains locked in a confined space and is unlocked and detached when in an open space. The detachment mechanism cannot be detached when in the confined space, and after being pushed out of the confined space, it is automatically unlocked and detached. However, this design disallows relocation of the mechanism through advancement or retraction once it has been detached. For an anatomically complex target site, this is unfavorable to accurate delivery.

Therefore, there is a need to develop a detachment mechanism and a delivery catheter, which can solve at least the above problems.

### SUMMARY OF THE INVENTION

It is an objective of the present invention to provide a detachment mechanism and a delivery catheter, which overcome the problems of disallowed relocation and insufficient delivery accuracy associated with existing detachment mechanisms.

To this end, the present invention provides a detachment mechanism for enabling delivery and detachment of an implant, which includes a detacher, a first control member and a second control member, a distal end of the detacher configured for engagement of the implant therewith, the detacher defining a through hole, the first control member and the second control member detachably engaged with or directly joined to each other, a distal end of the first control member and a distal end of the second control member configured to be inserted into the through hole, wherein when the distal end of the first control member and the distal end of the second control member are passed through the through hole and engaged with each other, the first control member and the second control member are locked against the detacher, and wherein when the first control member is pulled, the first control member is removed from the through hole, the second control member is unlocked from the detacher with the removal of the first control member, thereby detaching the first control member and the second control member from the detacher.

Optionally, the through hole of the detacher may include a straight hole and an interconnected hole in communication with the straight hole, wherein the distal end of the second control member is inserted in the interconnected hole, wherein the distal end of the first control member is inserted in the straight hole, and wherein the first control member and the second control member are detachably engaged with each other at a location where the interconnected hole comes into communication with the straight hole.

Optionally, the distal end of the second control member may define a control hole, wherein the first control member is passed through the control hole, thereby engaging the first and second control members with each other, and wherein when the first control member is removed from the control hole, the first and second control members are disengaged from each other.

Optionally, the first control member may include a control wire and an insertion pin, a distal end of the control wire coupled to a proximal end of the insertion pin, the insertion pin having a distal end configured to be inserted into the straight hole and locked to the second control member.

Optionally, an end portion of the distal end of the first control member and an end portion of the distal end of the second control member may be both passed through the through hole from the same one end to the other end, wherein a portion of the first control member that has not been passed through the through hole, a portion of the second control member that has not been passed through the through hole, a portion of the first control member that has been passed through the through hole from the other end, and a portion of the second control member that has been passed through the through hole from the other end are detachable in a controlled manner.

Optionally, the distal end of the first control member and the distal end of the second control member may be directly joined together.

Optionally, an end portion of the distal end of the first control member and an end portion of the distal end of the second control member may be directly joined together, wherein the end portion of the distal end of the first control member and the end portion of the distal end of the second control member are inserted into the through hole from opposite sides thereof and the first control member and the second control member are joined within the through hole.

Optionally, the through hole of the detacher may be a straight hole, an axis of the straight hole is perpendicular to an axis of the detachment mechanism, or not.

Optionally, the detacher may include a leading engagement portion and a trailing engagement portion, the leading engagement portion including a spherical structure, the leading engagement portion configured for engagement with the first and second control members, the trailing engagement portion defining a hollow hole configured for engagement of the implant.

Optionally, the detachment mechanism may further include a push tube, wherein the first and second control members are passed through the push tube and engaged with the detacher.

Optionally, the push tube may define a first lumen and a second lumen, which are configured for passage of the first control member and the second control member therethrough, respectively.

To the above end, the present invention also provides a delivery catheter including the detachment mechanism as defined above.

The present invention provides a detachment mechanism and a delivery catheter for enabling delivery and detachment of an implant. The detachment mechanism includes a detacher, a first control member and a second control member. A distal end of the detacher is configured for engagement of the implant therewith. The detacher defines a through hole. The first and second control members are detachably engaged with or directly joined to each other. Distal ends of the first and second control members are configured to be inserted into the through hole. When the distal ends of the first and second control members are inserted through the through hole and engaged with each other, the first and second control members are locked against the detacher. The first control member can be pulled and removed from the through hole, and in response, the second control member will be unlocked from the detacher, thereby detaching the first and second control members from the detacher. With this design, after the detacher is pushed out of a confined space, it is still under the control of the first and second control members and can be further advanced or retracted. Thus, this design allows relocation of the detacher, facilitates its detachment, satisfies high delivery and detachment requirements and enables delivery of the detacher with improved accuracy.

### BRIEF DESCRIPTION OF THE DRAWINGS

Those of ordinary skill in the art would appreciate that the accompanying drawings are provided to facilitate a better understanding of the present invention and do not limit the scope thereof in any sense, in which:
Fig. 1 is a schematic illustration of a delivery catheter according to a first embodiment of the present invention;
Fig. 2 is a schematic illustration of a detachment mechanism according to the first embodiment of the present invention;
Fig. 3 is a cross-sectional view of the detachment mechanism of Fig. 2;
Fig. 4 is a schematic illustration of a detacher in the detachment mechanism according to the first embodiment of the present invention;
Fig. 5 is a schematic illustration of an alternative detacher in the detachment mechanism according to the first embodiment of the present invention;
Fig. 6 is a schematic illustration of a push tube in the detachment mechanism according to the first embodiment of the present invention;
Fig. 7 is a schematic illustration of an alternative push tube in the detachment mechanism according to the first embodiment of the present invention;
Fig. 8 is a schematic illustration of a detachment mechanism according to a second embodiment of the present invention; and
Fig. 9 is a schematic illustration of a detachment mechanism according to a third embodiment of the present invention.

In these figures:
1-implant;
10-detacher; 11-through hole; 111-straight hole; 112-interconnected hole; 12-hollow hole;
20-first control member; 21-control wire; 22-insertion pin;
30-second control member; 31-control hole;
40-push tube; 41-first lumen; 42-second lumen;
50-sealing member;
60-outer tube;

A-first closed loop; B-second closed loop; C-third closed loop.

### DETAILED DESCRIPTION

Objectives, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, the structures shown in the figures are usually part of actual structures. In particular, as the figures tend to have distinct emphases, they are often drawn to different scales.

As used herein, the singular forms "a", "an" and "the" include plural referents, unless the context clearly dictates otherwise. As used herein, the term "or" is generally employed in the sense of "and/or", unless the context clearly dictates otherwise. Moreover, the term "several" is generally employed in the sense of "at least one", and "at least two" of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. The terms "mounting", "coupling" and "connection" should be interpreted in a broad sense. For example, a connection may be a permanent, detachable or integral connection, a direct or indirect connection with one or more intervening media, or an internal communication or interaction between two elements. As used herein, and the term "distal end" refers to an end farther away from an operating surgeon, and the term "proximal end" refers to an end closer to the operating surgeon. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location relative to the other element, unless the context clearly dictates otherwise. Those of ordinary skill in the art can understand the specific meanings of the above-mentioned terms herein, depending on their context. Additionally, the following description sets forth numerous specific details in order to provide a more thorough understanding of the present invention. However, it will be apparent to those skilled in the art that the present invention can be practiced without one or more of these specific details. In other instances, well-known technical features have not been described in order to avoid unnecessary obscuring of the invention.

The present invention provides a detachment mechanism and a delivery catheter for enabling delivery and detachment of an implant. The detachment mechanism includes a detacher, a first control member and a second control member. A distal end of the detacher is configured for engagement of the implant therewith. The detacher defines a through hole. The first and second control members are detachably engaged with or directly joined to each other. Distal ends of the first and second control members are configured to be inserted into the through hole. When the distal ends of the first and second control members are inserted through the through hole and engaged with each other, the first and second control members are locked against the detacher. The first control member can be pulled and removed from the through hole, and in response, the second control member will be unlocked from the detacher, thereby detaching the first and second control members from the detacher. With this design, after the detacher is pushed out of a confined space, it is still under the control of the first and second control members and can be further advanced or retracted. Thus, this design allows relocation of the detacher, facilitates its detachment, satisfies high delivery and detachment requirements and enables delivery of the detacher with improved accuracy.

The present invention will be further described below with reference to the accompanying drawings.

### EMBODIMENT 1

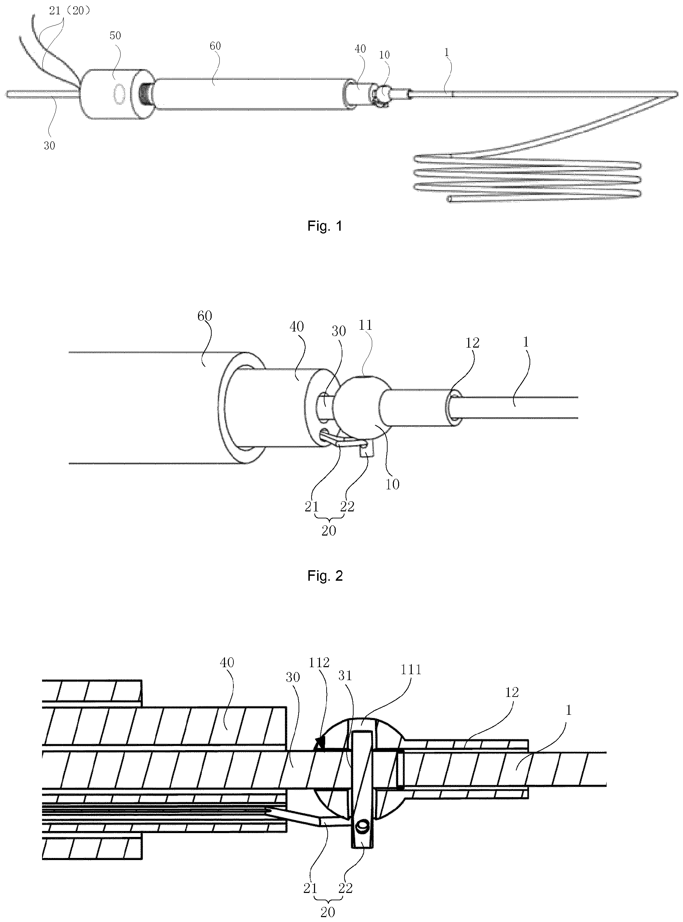

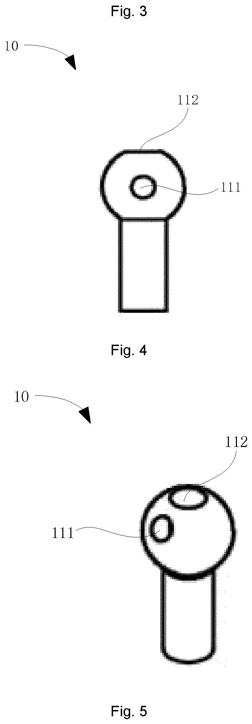

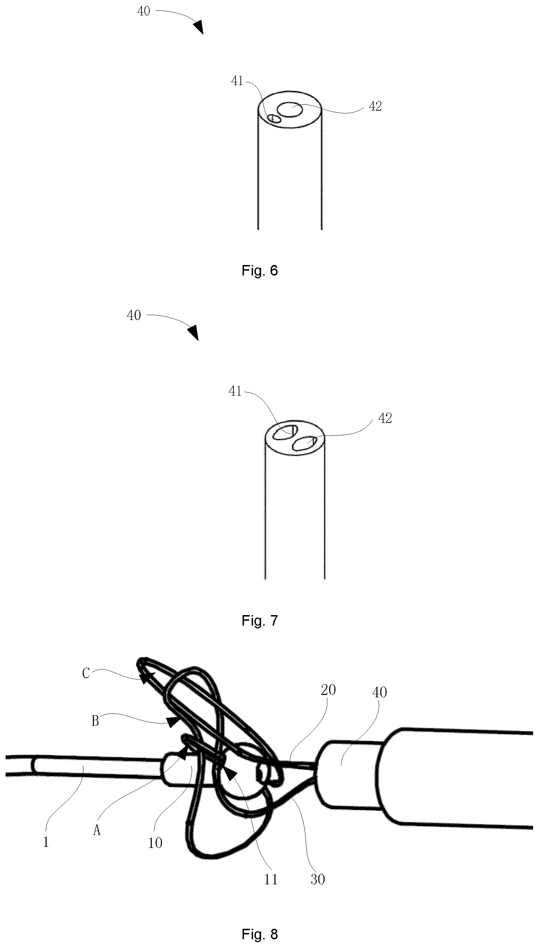

Reference is now made to Figs. 1 to 7. Fig. 1 is a schematic illustration of a delivery catheter according to a first embodiment of the present invention. Fig. 2 is a schematic illustration of a detachment mechanism in the first embodiment of the present invention. Fig. 3 is a cross-sectional view of the detachment mechanism of Fig. 2. Fig. 4 is a schematic illustration of a detacher in the detachment mechanism according to the first embodiment of the present invention. Fig. 5 is a schematic illustration of another detacher in the detachment mechanism according to the first embodiment of the present invention. Fig. 6 is a schematic illustration of a push tube in the detachment mechanism according to the first embodiment of the present invention. Fig. 7 is a schematic illustration of an alternative push tube in the detachment mechanism according to the first embodiment of the present invention.

In this embodiment, there is provided a detachment mechanism for enabling delivery and release of an implant 1, especially for enabling detachment of the implant 1. The detachment mechanism includes a detacher 10, a first control member 20 and a second control member 30.

A distal end of the detacher 10 is configured for engagement of the implant 1 therewith. It would be appreciated that, as used herein, the term "distal end" refers to an end farther away from an operating surgeon, and the term "proximal end" refers to an end closer to the operating surgeon. The implant 1 may be, for example, a coil anchor. The detacher 10 defines a through hole 11, the through hole 11 extends through the detacher 10, and a structural member can be inserted through the detacher 10. The detacher 10 includes a leading engagement portion and a trailing engagement portion. The leading engagement portion is preferred to include, for example, a spherical structure and is configured for engagement with the first control member 20 and the second control member 30. The trailing engagement portion defines a hollow hole 12, the hollow hole 12 is used for enabling engagement of the implant 1. For example, an engagement end of the coil anchor may be inserted into the hollow hole 12. The engagement end may be engaged with the hollow hole 12, for example, by mechanical locking, adhesive bonding or threaded engagement.

As shown in Fig. 2, distal ends of the first control member 20 and the second control member 30 are configured to be inserted in the through hole 11. The first control member 20 and the second control member 30 are detachably engaged with each other. As a result of the distal ends of the first control member 20 and the second control member 30 being inserted through the through hole 11 and engaged with each other, first control member 20 and the second control member 30 are locked to the detacher 10. When the first control member 20 is pulled out from the through hole 11, the second control member 30 will be consequently disengaged from the through hole 11, unlocking the first control member 20 and the second control member 30 from the detacher 10. The detachable engagement of the first control member 20 and the second control member 30 may be mechanical. For example, both the first control member 20 and the second control member 30 may be inserted in the detacher 10 and detachably engaged with each other there. More specifically, for example, the engagement of the first control member 20 and the second control member 30 may be accomplished by mutual entrapment. Further, for example, the distal end of the second control member 30 may define a control hole 31. Moreover, the first control member 20 may be inserted through the control hole 31 and thereby the first control member 20 is engaged with the second control member 30. Further, the first control member 20 may be pulled out of the control hole 31 and thereby the first control member 20 is disengaged from the second control member 30. Alternatively, the first control member 20 and the second control member 30 may be engaged with each other by a magnetic force. For example, a magnetic attractive force may be produced between the distal ends of the first control member 20 and the second control member 30 when an electrical signal is applied thereto, thereby engaging them to each other. In this case, simply by removing the electrical signal, the first control member 20 and the second control member 30 may be disengaged from each other, detaching the detacher 10. With this design, after the detacher 10 is pushed out of a confined space, it is still under the control of the first control member 20 and the second control member 30 and can be further advanced or retracted. Thus, this design allows relocation of the detacher 10, facilitates its detachment and has particular utility in attachment and detachment of miniature structures. It satisfies high delivery and detachment requirements and enables delivery of the detacher 10 with improved accuracy. The detachment mechanism of this embodiment does not rely on complex mechanical locking and is easy to fabricate and operate. The confined space for the detacher 10 may be defined by an outer tube 60 of a catheter. More preferably, each of the first control member 20, the second control member 30 and the detacher 10 is made of a plastic material, which can enhance biocompatibility.

Preferably, as shown in Fig. 3, the through hole 11 of the detacher 10 includes a straight hole 111 and an interconnected hole 112 in communication with the straight hole 111. The distal end of the second control member 30 is inserted in the interconnected hole 112, and the distal end of the first control member 20 is inserted in the straight hole 111. The first control member 20 and the second control member 30 are detachably engaged with each other at a location where the straight hole 111 and the interconnected hole 112 come into communication with each other. For example, the second control member 30 may be a rigid guide wire. For example, the first control member 20 may be a flexible guide wire. For example, the first control member 20 may be passed through the distal end of the second control member 30 and thereby restrict the second control member 30 within the detacher 10. Alternatively, the first control member 20 may be wound on the distal end of the second control member 30 and thereby similarly restrict the second control member 30 within the detacher 10. When the first control member 20 is pulled and the first control member 20 is disengaged from the second control member 30, the engagement of the first control member 20 and the second control member 30 with the detacher 10 is also destroyed. The straight hole 111 and the interconnected hole 112 are configured to allow disengagement of the first control member 20 and the second control member 30 from each other within the detacher 10.

More preferably, the distal end of the second control member 30 defines a control hole 31, and the first control member 20 may be inserted through the control hole 31 and thereby the first control member 20 is engaged with the second control member 30. This allows the first control member 20 to restrict the second control member 30. Moreover, the first control member 20 may be removed from the control hole 31 and thereby the first control member 20 is disengaged from the second control member 30, destroying the engagement of the first control member 20 and the second control member 30 with the detacher 10. Specifically, in this embodiment, the first control member 20 is preferred to include a control wire 21 and an insertion pin 22. A distal end of the control wire 21 is coupled to a proximal end of the insertion pin 22, and a distal end of the insertion pin 22 is configured to be inserted into the straight hole 111 and locked to the second control member 30. Of course, in other embodiments, a proximal end of the first control member 20 may define a through hole, and the second control member 30 may be inserted into the through hole of the first control member 20. In this way, the first control member 20 may be locked with the second control member 30.

Preferably, the through hole 11 of the detacher 10 is a straight hole 111, an axis of the straight hole 111 is perpendicular to an axis of the detachment mechanism or not. Moreover, the detacher 10 includes a spherical structure. Specifically, the leading engagement portion of the detacher 10 may be the spherical structure, which allows the detacher 10 to be compatible with human tissue. The trailing engagement portion of the detacher 10 is configured for engagement of an implant 1 therewith. The detacher 10 is generally cylindrical, making the trailing engagement portion well compatible with human tissue. In the embodiment shown in Fig. 4, the axis of the straight hole 111 in the detacher 10 is perpendicular to the axis of the detachment mechanism. For example, the axis of the detachment mechanism may define a direction in which a push tube 40 in the detachment mechanism extends. The first control member 20 may be inserted through the straight hole 111. Of course, in other embodiments, for example, in the second and third embodiments described below, as shown in Fig. 5, the axis of the straight hole 111 in the detacher 10 may be not perpendicular to the axis of the detachment mechanism. Thus, the engagement of the first control member 20 and the second control member 30 can be established in the detacher 10 in various manners.

Preferably, as shown in Figs. 6 and 7, the detachment mechanism further includes a push tube 40, and the first control member 20 and the second control member 30 are inserted through the push tube 40 and then engaged with the detacher 10. Specifically, the push tube 40 may define a through lumen for passage of the first control member 20 and the second control member 30 therethrough. This can facilitate engagement of the first control member 20 and the second control member 30 with the detacher 10. The push tube 40 may define one, two or more through lumens. Preferably, there are two through lumens, referred to hereinafter respectively as a first lumen 41 and a second lumen 42, which are configured for passage of the first control member 20 and the second control member 30 therethrough, respectively. For example, the first control member 20 may be passed through the first lumen 41, and the second control member 30 may be passed through the second lumen 42. The first lumen 41 and the second lumen 42 may have equal diameters, or not. Preferably, the diameters of the first lumen 41 and the second lumen 42 may be determined according to those of the first control member 20 and the second control member 30 inserted therethrough. This dual lumen design can prevent the first control member 20 and the second control member 30 from rubbing against each other or tangling with each other and facilitate their retraction, advancement and detachment.

In this embodiment, there is also provided a delivery catheter including the detachment mechanism as defined above. Preferably, the delivery catheter further includes an outer tube 60, and the push tube 40 in the detachment mechanism is inserted through the outer tube 60. The delivery catheter has all the advantages of the detachment mechanism, and further description thereof is deemed unnecessary. The delivery catheter may further include other conventional components, the operation of which is known in the art and, therefore, needs not be described in further detail herein.

### EMBODIMENT 2

Reference is now made to Fig. 8, Fig. 8 is a schematic illustration of a detachment mechanism according to a second embodiment of the present invention.

Any common feature of the second embodiment shared with the first embodiment will not be repeated here, and only differences between these two embodiments will be described in detail below.

As shown in Fig. 8, the detachment mechanism according to this embodiment includes a detacher 10, a first control member 20 and a second control member 30.

The end portion of the distal end of the first control member 20 and the end portion of the distal end of the second control member 30 are both inserted through a through hole 11 from the same one end to the other. Portions of the first control member 20 and the second control member 30 that have not been passed through the through hole 11 yet (referred to herein after as "non-passed portions") and their portions that have been passed through and out of the through hole 11 are detachable in a controlled manner ("passed portions"). Here, by "detachable in a controlled manner", it is intended to mean that those portions are engaged together, preferably by a releasable knot. For example, the first control member 20 and the second control member 30 may be separate wires. Preferably, the first control member 20 and the second control member 30 may be directly joined together at their distal ends. In this case, the first control member 20 and the second control member 30 may be considered as different portions of a single wire. In the embodiment of Fig. 8, the distal end of the first control member 20 and the second control member 30 are directly joined together, and forms a first closed loop A on the other side of the through hole 11 in the detacher 10. The non-passed portion of the second control member 30 is passed through the first closed loop A from one side to the other and thereby forms a second closed loop B. The first control member 20 may be pulled back to tie the second closed loop B in the first closed loop A. The non-passed portion of the first control member 20 is passed through the second closed loop B from one side to the other and thereby forms a third closed loop C. The second control member 30 may be pulled back to tie the third closed loop C in the second closed loop B. During delivery, the wire of the second control member 30 may be tightened to engage a push tube 40 with the detacher 10 without detachment during advancement or retraction. In order to detach the push tube 40 and the detacher 10 from each other, the wire of the first control member 20 may be pulled to untie the third closed loop C, and the second closed loop B and the first closed loop A may be then successively untied in a similar manner, accomplishing the detachment. Preferably, the push tube 40 may define a first lumen 41 and a second lumen 42, which can separate the first control member 20 and the second control member 30 from each other in space, avoiding them from rubbing against or tangling with each other. This can facilitate detachment and increase delivery reliability. Preferably, the through hole 11 is a straight hole 111. More preferably, an axis of the through hole 11 is angled with respect to an axis of the detachment mechanism, and the other side of the through hole 11 faces away from the push tube. Of course, instead of being directly joined together, the first control member 20 and the second control member 30 may also be provided as separate wires. In this case, both wires may be passed through the through hole 11 and tied to each other on the other side of the through hole 11 with a releasable knot, which allows easy engagement and disengagement of the detacher 10. With this design, after the detacher 10 is pushed out of a confined space, it is still under the control of the first control member 20 and the second control member 30 and can be further advanced or retracted. Thus, this design allows relocation of the detacher 10 and increases its delivery accuracy.

### EMBODIMENT 3

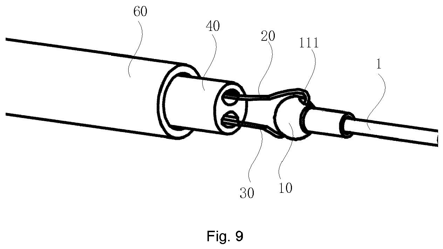

Reference is now made to Fig. 9, Fig. 9 is a schematic illustration of a detachment mechanism according to a third embodiment of the present invention.

Any common feature of the third embodiment shared with the first or second embodiment will not be repeated here, and only its differences from the two embodiments will be described in detail below.

As shown in Fig. 9, the detachment mechanism according to this embodiment includes a detacher 10, a first control member 20 and a second control member 30.

The end portion of the distal end of the first control member 20 and the end portion of the distal end of the second control member 30 are directly joined together. The end portion of the distal end of the first control member 20 and the end portion of the distal end of the second control member 30 are inserted into the through hole 11 from its opposite sides and joined within the through hole 11. This allows the first control member 20 and the second control member 30 to control detachment of the detacher 10. For example, the first control member 20 and the second control member 30 may be provided as separate wires, but since they are joined together, the first control member 20 and the second control member 30 may be considered as a single wire. It would be appreciated that the wire made up of the first control member 20 and the second control member 30 is passed through the through hole 11 from one side to the other, thereby locking the detacher 10. Its detachment can be achieved by pulling a proximal end of the first control member 20 or the second control member 30 to remove the first control member 20 and the second control member 30 from the through hole 11. Preferably, the through hole 11 is a straight hole 111. More preferably, an axis of the through hole 11 is angled with respect to an axis of the detachment mechanism. It would be appreciated that the proximal ends of the first control member 20 and the second control member 30 are locked to a sealing member 50 provided at a proximal end of the detachment mechanism. Specifically, the sealing member 50 may be, for example, provided at a proximal end of a push tube 40 and configured to lock the first control member 20 and the second control member 30. Before the detachment, the proximal ends of the first control member 20 and the second control member 30 may be both unlocked from the sealing member 50. One of the proximal ends of the first control member 20 and the second control member 30 may be then pulled to cause the other end to move distally and then leave the detacher 10. During delivery, the push tube 40 may directly contact the detacher 10 and push it forwards. In order to move, e.g., retract, the detacher 10, the first control member 20 and the second control member 30 may be both held and pulled back. With this design, after the detacher 10 is pushed out of a confined space, it is still under the control of the first control member 20 and the second control member 30 and can be further advanced or retracted. Thus, this design allows relocation of the detacher 10 and increases its delivery accuracy.

In summary, the present invention provides a detachment mechanism and a delivery catheter for enabling delivery and detachment of an implant. The detachment mechanism includes a detacher, a first control member and a second control member. A distal end of the detacher is configured for engagement of the implant therewith. The detacher defines a through hole. The first and second control members are detachably engaged with or directly joined to each other. Distal ends of the first and second control members are configured to be inserted into the through hole. When the distal ends of the first and second control members are inserted through the through hole and engaged with each other, the first and second control members are locked against the detacher. The first control member can be pulled and removed from the through hole, and in response, the second control member will be unlocked from the detacher, thereby detaching the first and second control members from the detacher. With this design, after the detacher is pushed out of a confined space, it is still under the control of the first and second control members and can be further advanced or retracted. Thus, this design allows relocation of the detacher, facilitates its detachment, satisfies high delivery and detachment requirements and enables delivery of the detacher with improved accuracy.

It is to be noted that the description of each embodiment focuses on its differences from others, and reference can be made between the embodiments for their common or similar features.

Further, it is to be recognized that while the invention has been described above with reference to preferred embodiments thereof, it is not intended to be limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. A detachment mechanism for enabling delivery and detachment of an implant, comprising a detacher, a first control member and a second control member,
a distal end of the detacher configured for engagement of the implant therewith,
the detacher defining a through hole, the first control member and the second control member detachably engaged with or directly joined to each other, a distal end of the first control member and a distal end of the second control member configured to be inserted into the through hole,
wherein when the distal end of the first control member and the distal end of the second control member are passed through the through hole and engaged with each other, the first control member and the second control member are locked against the detacher, and wherein when the first control member is pulled, the first control member is removed from the through hole, the second control member is unlocked from the detacher with the removal of the first control member, thereby detaching the first control member and the second control member from the detacher.

2. The detachment mechanism according to claim 1, wherein the through hole of the detacher comprises a straight hole and an interconnected hole in communication with the straight hole, wherein the distal end of the second control member is inserted in the interconnected hole, wherein the distal end of the first control member is inserted in the straight hole, and wherein the first control member and the second control member are detachably engaged with each other at a location where the interconnected hole comes into communication with the straight hole.

3. The detachment mechanism according to claim 2, wherein the distal end of the second control member defines a control hole, wherein the first control member is passed through the control hole, thereby engaging the first control member and the second control member with each other, and wherein when the first control member is removed from the control hole, the first control member and the second control member are disengaged from each other.

4. The detachment mechanism according to claim 3, wherein the first control member comprises a control wire and an insertion pin, a distal end of the control wire coupled to a proximal end of the insertion pin, the insertion pin having a distal end configured to be inserted into the straight hole and locked to the second control member.

5. The detachment mechanism according to claim 1, wherein an end portion of the distal end of the first control member and an end portion of the distal end of the second control member are both passed through the through hole from the same one end to the other end, wherein a portion of the first control member that has not been passed through the through hole, a portion of the second control member that has not been passed through the through hole, a portion of the first control member that has been passed through the through hole from the other end, and a portion of the second control member that has been passed through the through hole from the other end are detachable in a controlled manner.

6. The detachment mechanism according to claim 5, wherein the distal end of the first control member and the distal end of the second control member are directly joined together.

7. The detachment mechanism according to claim 1, wherein an end portion of the distal end of the first control member and an end portion of the distal end of the second control member are directly joined together, wherein the end portion of the distal end of the first control member and the end portion of the distal end of the second control member are inserted into the through hole from opposite sides thereof and the first control member and the second control member are joined within the through hole.

8. The detachment mechanism according to claim 1, wherein the through hole of the detacher is a straight hole, an axis of the straight hole is perpendicular to an axis of the detachment mechanism.

9. The detachment mechanism according to claim 1, wherein the through hole of the detacher is a straight hole, an axis of the straight hole is not perpendicular to an axis of the detachment mechanism.

10. The detachment mechanism according to claim 1, wherein the detacher comprises a leading engagement portion and a trailing engagement portion, the leading engagement portion comprising a spherical structure, the leading engagement portion configured for engagement with the first control member and the second control member, the trailing engagement portion defining a hollow hole configured for engagement of the implant.

11. The detachment mechanism according to claim 1, wherein the detachment mechanism further comprises a push tube, and wherein the first control member and the second control member are passed through the push tube and engaged with the detacher.

12. The detachment mechanism according to claim 11, wherein the push tube defines a first lumen and a second lumen, which are configured for passage of the first control member and the second control member therethrough, respectively.

13. The detachment mechanism according to claim 1, wherein the first control member is a flexible guide wire, wherein the second control member is a rigid guide wire, and wherein the first control member is wound on the distal end of the second control member.

14. The detachment mechanism according to claim 1, wherein a proximal end of the first control member and a proximal end of the second control member are locked to a sealing member provided at a proximal end of the detachment mechanism.

15. A delivery catheter, comprising the detachment mechanism according to any one of claims 1 to 14.
